Europäisches Patentamt

European Patent Office

Office européen des brevets

(11) Publication number: **0 580 252 A2**

## (12) EUROPEAN PATENT APPLICATION

(21) Application number: **93202152.0**

(22) Date of filing: **13.07.93**

(51) Int. Cl.5: **C12N 9/18**

(30) Priority: **20.07.92 EP 92306636**

(43) Date of publication of application:
**26.01.94 Bulletin 94/04**

(84) Designated Contracting States:
**AT BE CH DE DK ES FR GB IE IT LI NL SE**

(71) Applicant: **OUEST INTERNATIONAL B.V.**
**Huizerstraatweg 28**
**NL-1411 GP Naarden(NL)**

(72) Inventor: **Cheetham, Peter Samuel James**
**Unilever Research Colworth Lab.,**
**Colworth House**
**Sharnbrook, Bedford MK44 1LO(GB)**
Inventor: **Hauton, David**
**Unilever Research Colworth Lab.,**
**Colworth House**
**Sharnbrook, Bedford MK44 1LO(GB)**

(74) Representative: **Dekker, Enno E.J. et al**
**Unilever N.V.**
**Patents Division**
**P.O. Box 137**
**NL-3130 AC Vlaardingen (NL)**

(54) **Improvements in or relating to pectin methyl esterase.**

(57) A novel class of pectin methyl esterases, having pectin methyl esterase activity not inhibited by tyrosine selective inhibitors, is disclosed. The enzymes are present in certain plant enzyme extracts, particularly papain, ficin, bromelain and wheat germ lipase. The enzymes are effective to reduce the degree of methoxylation of pectin without significant reduction in pectin molecular weight.

EP 0 580 252 A2

Rank Xerox (UK) Business Services
(3.10/3.09/3.3.4)

Field of the invention

This invention relates to pectin methyl esterase (referred to herein as "PME" for brevity) and concerns novel PMEs and their use in demethoxylating pectin.

Background to the invention

Pectin is a polysaccharide, isolated from the cell wall material of plants. It is a macromolecule consisting of a backbone of partially methoxylated polygalacturonic acid. The polysaccharide is built up from D galacturonic acid units bonded by alpha 1 - 4 glycosidic linkages. The structure has several branch points where additional neutral sugars are bonded to the polygalacturonic acid backbone in the form of ethers (in the 2 and 3 positions). These additional sugars are predominantly rhamnose, galactose, arabinose and xylose, and the resulting side chains are known as "hairy" regions.

Pectin is commercially available in three forms: high methoxy pectin, amidated pectin and low methoxy pectin. In high methoxy pectin greater than 50% of the galacturonic acid is in the form of galacturonic acid methyl ester, whereas in low methoxy pectin less than 50% (typically 30-40%) of the galacturonic acid is in the esterified form. In amidated pectin de-esterification has been carried out with ammonium hydroxide which derivatises the pectin forming -CONH$_2$ groups.

The food industry uses both high and low methoxy forms of pectin extensively, e.g. as a gelling agent for jam and jelly, and also as an emulsifier or thickener. High methoxy pectin combined with sugar under acid conditions forms a gel when heated and then allowed to cool. However, low methoxy pectin does not require the use of sugar and acid, and will gel in the presence of divalent ions (e.g. Ca$^{2+}$) to produce thermostable gels which are suitable for use in, e.g., dairy-based products, diet foods and diabetic preparations. Low methoxy pectin is also found to have less adverse effect on the flavour of food products such as jams: see the article by Guichard et al in Journal of Food Science, Volume 56, No.6, 1991, 1621-1627.

Gellation of high methoxy pectin occurs by association of chains, stacking the esterified galacturonic acid regions together: these junctions are stabilised by hydrogen bonding which is induced by the reduction in water activity (caused by the addition of sugars). Acidification reduces the electrostatic repulsions and therefore improves gelling characteristics. This favours hydrophobic interactions between methyl ester groups.

Low methoxy pectin is thought to gel in the presence of divalent ions by the "egg box model": this does not involve true electrostatic attractions, but instead is due to true intermolecular chelation leading to zones of aggregation of the polysaccharide, which form between non-esterified galacturonic acid residues.

Pectin obtained from natural plant sources such as apple and citrus fruits is generally in the form of high methoxy pectin, typically having a degree of methoxylation (DM or DE) of about 70%.

There are a number of ways in which high methoxy pectin can be treated to reduce the degree of methoxylation, including the following:

1) Treatment with sodium hydroxide. However, such treatment also causes some breakage of glycosidic bonds in the pectin backbone, resulting in production of lower molecular weight pectin with reduced functional properties. In addition much of the neutral sugars present in the hairy regions of the pectin are lost.

2) Treatment with acid. As with sodium hydroxide treatment, this also results in breakage of glycosidic bonds in the pectin backbone, producing lower molecular weight pectin with reduced functional properties.

3) Treatment with ammonium hydroxide, producing amidated pectin as mentioned above. However, this form of pectin is becoming increasingly less preferred by consumers and regulatory authorities.

4) Treatment with pectin methyl esterases. PMEs act by hydrolysing the methoxy groups of pectin to produce low methoxy pectins without breakage of glycosidic bonds in the pectin backbone. PMEs are obtainable from plant sources such as tomato and orange and from bacterial sources: see, e.g. US Patent 4200694. However, PMEs are expensive and are not currently available in forms suitable for use in foods. Normally they occur as components of pectinases which are complex mixtures of different enzyme activities including pectin and pectate lyases and polygalacaturonidase which break the pectin down into low molecular weight non-functional oligosaccharides.

As a result of investigations to find enzymes with good methyl esterase activity, no polymer cleavage activity and that are readily and cheaply available and suitable for food use, a novel class of PMEs has been discovered.

## Summary of the invention

In one aspect the present invention provides an enzyme having pectin methyl esterase activity not inhibited by tyrosine selective inhibitors.

It can be inferred from the inhibition behaviour that a PME in accordance with the invention does not have a tyrosine residue at the enzyme active site, and so is distinguished from known plant PMEs such as orange and tomato PME which do have a tyrosine residue at the enzyme active site and are inhibited by tyrosine selective inhibitors such as N-acetyl imidazole.

Novel PMEs in accordance with the invention have been obtained from plant sources, being found to be present in certain plant enzyme extracts, particularly papain, ficin, wheat germ lipase (WGL), bromelain etc.

In addition to the inhibition behaviour regarding tyrosine, the PME's according to the invention are not inhibited by a cysteine selective inhibitor. Papain and ficin derived PME are also not inhibited by a serine selective inhibitor.

Papain, ficin and bromelain are cysteine proteases present in papaya, fig and bromelard sap respectively, and WGL is a lipase present in wheat germ. Investigations by the present inventors have shown that commercially available preparations of papain, ficin and WGL have distinct PME activity in addition to the expected protease and lipase activity, respectively. Purification experiments have shown that the commercially available preparations, hitherto thought to have either only protease or lipase activites, are in fact impure and consist of a mixture of protease or lipase (having no PME activity), together with a separate enzyme having PME activity. The PMEs of the invention thus constitute previously undiscovered contaminating enzymes in plant enzyme extracts such as papain, ficin and WGL. It is possible that other plant enzyme extracts, e.g. from kiwi fruit, pineapples etc., will also be found to contain novel PMEs in accordance with the invention.

It is surprisingly found that these plant enzyme extracts (unlike other known plant PMEs) contain high PME activity but are lacking in significant glycosidase activity such as polygalacturonidase and pectin and pectate lyases that usually accompany them. In particular it has been found that very impure sources of the enzymes contain much higher PME activites, but still apparently no significant pectin backbone degrading enzyme activity.

In addition, the PMEs of the invention, obtained from papain and ficin at least, are stable to cysteine proteases (presumably due to the natural coexistence of the enzymes), whereas known tomato PME is degraded and inactivated by cysteine proteases. By way of illustration, PMEs present in papain and ficin show no significant loss of activity even after being incubated with papain for about 100 hours at a temperature of 50°C and at a pH 5.5. In contrast, tomato PME loses a lot of activity even over a shorter period when incubated under the same conditions. Sodium dodecyl sulphate-,polyacrylamide gel electrophoresis (SDS-PAGE) indicates the novel PMEs of the invention have molecular weights of about 58,000 (papain) to 61,000 (ficin), which is comparable to the molecular weight of known plant PMEs such as orange PME (58,000). However, the novel PMEs of the invention are distinguished from known plant PMEs by inhibition behaviour, as discussed above.

PMEs in accordance with the invention are obtainable by isolation from plant sources, e.g. using conventional enzyme purification techniques. Alternatively the PMEs of the invention can be produced by genetic engineering techniques, by isolating and cloning the gene coding for the protein of interest and introducing the gene into a suitable host, e.g. yeast or bacterial host, for expression and cost-effective large scale production.

The novel PMEs of the invention find application in reducing the degree of methoxylation of pectin, particularly in demethoxylation of high methoxy pectins to produce low methoxy pectin, without significant reduction in pectin backbone length.

Thus, in a further aspect, the present invention provides a method of reducing the degree of methoxylation of pectin, comprising treating pectin with a PME in accordance with the invention.

Purified PMEs in accordance with the invention can be used for this purpose. However, good results have been obtained by using commercially available preparations of ficin (EC 3.4.22.3), papain (EC 3.4.22.2), bromelain (EC 3.4.22.4) and WGL (EC 3.1.1.3) in which PME is present as a hitherto unappreciated contaminant. Use of such preparations has the advantage that the preparations are commercially available, are relatively cheap, and are suitable for food use, being already classified as "generally recognised as safe for use in goods" (GRAS) enzymes. Cruder (and cheaper) enzyme sources such as papaya and ficin latex, which are very crude, impure materials, have been found to include greater PME activity and to be well suited to use in the method of the invention. In fact, crude latex from papaya may have a higher PME activity per gram when compared with purified papain. For some purposes, it can therefore be quite attractive to use crude latex from papaya instead of purified papain. It is thought the

3

proteases present in papain and ficin may act to degrade any contaminating protein in the pectin, providing an added advantage. However, use of crude enzyme sources has the disadvantage of requiring a large amount of enzyme solids. In practice, it might therefore be commercially desirable to purify PME in accordance with the invention from papaya or ficin latex, e.g. by suitable modification of convention purification processes designed to produce papin and ficin, to obtain concentrated sources of PME in accordance with the invention in addition to papain and ficin.

The PME activities of various enzyme preparations are given in Table 1, which also demonstrates that pure papain, ficin and bromelain preparations have no PME activity.

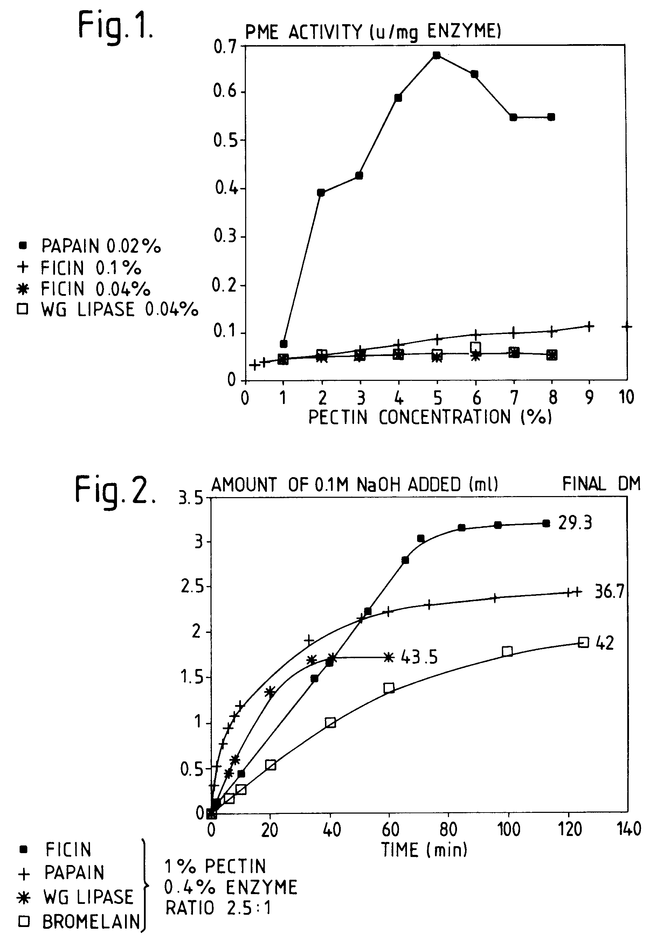

Enzyme activity varies with pectin concentration, as illustrated in Figure 1.

Under suitable conditions, PMEs in accordance with the invention can have PME activities comparable to those of known plant PMES, with similar reaction rates.

PMEs in accordance with the invention are found to be effective at demethoxylating pectins having a wide range of degrees of methoxylation, ranging down from about 70%, so use of PMEs in accordance with the invention can produce pectins with a very low degree of methoxylation and lower than has hitherto been commercially possible (30%-40%). Papain has a maximum reaction rate with pectin at DM of 40%. For ficin although the initial rates of reaction did not show a big variation with the degree of methoxylation of the substrate, the activity expressed over the full time course of the reaction was much higher using high (60%) rather than low (27%) methoxy substrate. This effect was because ficin has a much greater catalytic rate on 60% DM substrate, as compared to 27% DM substrate (Vmax of 5.85 and 1.33, respectively), despite the enzyme actually having a higher affinity for the lower DM substrate ($K_m$ of 3.5 and 1.01 mgl$^{-1}$, respectively). $K_m$ data of various enzymes are given in Table 2.

PMEs in accordance with the invention are effective at demethoxylating pure pectin and also impure (cheaper) pectin, e.g. from apple and citrus sources, with ficin in some cases giving better results than papain on impure pectins. In some cases, when using ficin and papain rates of reaction are slightly higher on impure pectins than on pure pectins, possibly because the protease activity helps degrade contaminating proteins.

It is preferred that the pectin is at relatively low concentrations typically about 10% (w/v) or less so that the substrate is in solution and has a relatively low viscosity for ease of handling.

Papain PME is found to be maximally active with pectin at a concentrated 5% (w/v), while ficin and WGL PMEs are maximally active with 1% (w/v) pectin. Papain activity on 1% pectin is inhibited by methanol and stimulated by sodium chloride, but both these effects are hardly noticed on reactions with 5% pectin. The effects of methanol and sodium chloride on papain PME are illustrated in Table 3.

The degree of demethoxylation of pectin is found to be proportional to the amount of PME added. It is therefore possible to control the extent of demethoxylation of a given reaction simply by varying the amount of enzyme added; the more PME added, the faster the rate of reaction and the lower the final DM of the pectin.

Ficin and papin are both found to have optimum PME activity at a pH of about 8, with the temperature optimum for ficin being 60°C and that for papain being 50°C. Known plant PMEs have similar pH optima, but generally do not survive temperatures above about 40°C. It may therefore be possible to carry out demethoxylation of pectin using PMEs of the invention at higher temperatures than have hitherto been used, with greater efficiency in processing. The reaction nevertheless proceeds well at lower temperatures, e.g. 20°C, although less rapidly.

For efficient functioning of the process it is important to maintain pH at or near the optimum value. Carboxyl groups are formed on the pectin as a result of the demethoxylation reaction, and it is therefore necessary to take steps to maintain pH levels. This is conveniently achieved by adding alkali, e.g. NaOH. It is preferred that this is achieved by adding an alkaline aqueous solution at a suitable rate to maintain a pH of between 6 and 9. It is found best results are obtained by adding 0.1M NaOH. This is in preference to 1.0M NaOH in preference in turn to 0.2M NaOH, in preference in turn to 0.01M NaOH. 0.1M NaOH is found to represent a compromise between using alkali that is too dilute and so not strong enough to restore pH rapidly, especially when the reaction is proceeding rapidly, with the added disadvantage of diluting the substrate, and using alkali that is too concentrated and can inactive the PME. After reaction, a desalting step may be carried out to remove surplus salt, followed by a drying step if required. Drying is conveniently achieved by heating, which will also have the effect of deactivating enzymes.

Reaction times are not critical but depend on conditions, and are typically between 60 and 200 minutes, depending on conditions such as the concentrations of pectin and enzyme used, the temperature and the efficiency with which pH is maintained.

The $K_m$ value of ficin PME is quite similar to that of known plant PMEs, while papain PME has a higher $K_m$ value than known plant PMEs.

Work has been carried out to examine the complete time course of the PME-pectin reaction of the invention. PME sources (ficin, papain, bromelain and WGL) have been compared, which has shown that the initial rate activities of the enzymes and the final DM values of the pectins produced are quite different. Also, the enzyme that starts off most actively does not necessarily produce pectins with lower DM values. The PME initial rate activity of papain is greater than that of WGL which in turn is equivalent to that of ficin. However, the final DM values obtained were lowest for ficin, followed by papain, then bromelain, with highest DM values for WGL, as illustrated in Figure 2. Addition of fresh substrate at the end of the reaction starts the reaction again, showing that active enzyme still remains and that the initial reaction finished because the enzyme could no longer act on the lower DM pectins it had already produced. This was confirmed since addition of ficin, which can react to form the lowest DM pectins, at the end of a papain catalysed reaction also reinitiated the reaction. Addition of NaCl significantly accelerated the initial rate of reaction, but actually led to the formation of pectins of rather higher DM than in the absence of NaCl, perhaps because the sodium salt of low methoxy pectin is not a good substrate for the PME. In each case the low methoxy pectin formed gelled readily when $CaCl_2$ was added.

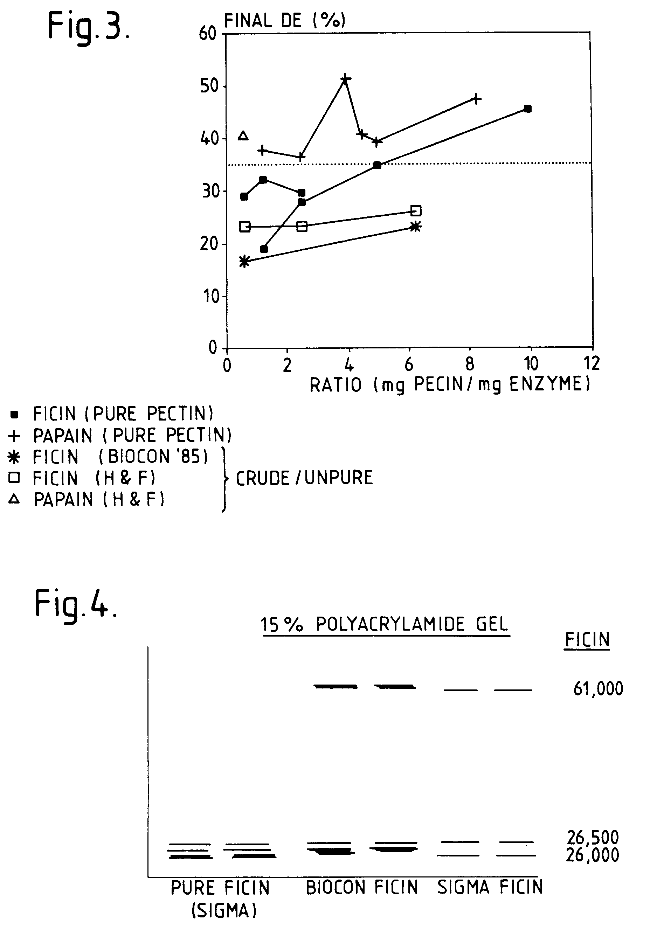

The final DM achieved also depends on the relative proportions of pectin and enzyme used, as is illustrated in Figure 3. See also Table 7. It is significant that ficin reproducibly forms lower final DM low methoxy pectins than does papain under comparable conditions, presumably because the papain is more easily inhibited by the methanol produced. Impure pectins appear to form low methoxy pectins of lower final DM than when purified pectin substrates are used, despite the original impure pectins having generally higher starting DMs. Use of the most active crude enzyme enabled LMPs to be produced using either ficin or papain. Papain is more readily available and cheaper and so may prove to be the most cost-effective option, but ficin can form significantly lower DM pectins and so may be preferred when these are required, perhaps being used to "finish" a papain reaction.

Unlike ficin PME and other known plant PMEs, papain PME under certain conditions is inhibited by methanol (a product of the demethoxylation reaction). None of the PMEs is inhibited by galacturonic acid even up to concentrations of 25 $gl^{-1}$.

A comparison of properties of different PMEs is given in Table 4.

Pectin treated by the method of the invention is found to have reduced methoxy content, possibly very low methoxy content down to about 5%, and so to be capable of gelling in the presence of calcium ions, without significant loss of molecular weight due to pectin backbone cleavage. Such low methoxy pectin, when produced by use of GRAS enzymes such as ficin and papain, is well suited to use in the food industry.

It is also thought that pectin treated by the method of the invention retains a large proportion of the hairy side chains (or neutral sugar side chains), so that the resulting pectin can be distinguished from chemically produced low methoxy pectins in which the majority of the hairy regions are lost. The invention thus provides a low methoxy, high molecular weight pectin having a neutral sugar side chains. Preferably, at least 60%, more preferably at least 70%, even more preferably at least 80% and most preferably at least 90% of the neutral sugar side chains is retained when treating a high methoxy pectin (having 100% neutral sugar side chains) with an enzyme according to the invention when compared with low methoxy pectin produced by e.g. NaOH treatment. This is exmplified by a simple test in which neutral sugars including rhamnose, arabinos, galactose, glucose, etc. were analysed by Dionex hplc analysis following hydrolysis of the pectin. Indexing the original gum neutral sugar content at 100%, papain PME and ficin PME-treated samples retained 93.2 and 81.5 % of the neutral sugars, but low methoxy pectin produced by NaOH treatment instead retained only 24.1% neutral sugars.

In a further aspect the present invention also provides a low methoxy pectin, produced by treatment of pectin with PME in accordance with the invention.

The invention thus potentially enables cheap and readily available crude pectin sources, e.g. apple pectin, to be processed to produce low methoxy pectins with properties (e.g. molecular weight and degree of esterification) equivalent to, or superior to, premium low methoxy pectins currently produced by chemical treatment of top quality expensive pectin raw materials, e.g. lime pectin.

The invention will be further described, by way of illustration, in the following description and Examples and with reference to the accompanying drawings, in which:

Figure 1 is a graph of PME activity (U/mg enzyme) versus pectin concentration (%);

Figure 2 is a graph of time in minutes versus amount of 0.1M NaOH added in ml, illustrating profiles of the demethoxylation of pectin using 1% pectin and 0.4% enzyme (i.e. ratio of 2.5:1), and showing final DM values;

Figure 3 is a graph of ratio of weight of pectin to enzyme versus final DM, showing results obtained using impure apple pectin and also data obtained using pure pectin;

Figure 4 is a schematic diagram of the results of SDS PAGE of ficin preparations;

Figure 5 is a schematic diagram of the results of SDS PAGE of papain preparations; and

Figure 6 is a graph of reaction time in minutes versus volume of 0.1M NaOH added in mls, showing the activity of Arinor papain, with and without NaCl, on semipurified pectin.

## CM-Sepharose Batch Separation of Protease

### Components of Ficin

Separation was achieved using an adapted version of a method of ficin purification (Hussain S.S.and Lowe G. (1970) Biochem. J. 117, 333-340). 10gms of ficin powder was dissolved in 100mls of distilled water and raised to pH = 7.1 (0.1M NaOH). This solution was then combined with 100mls of CM-Sepharose (pH = 7.1, 0.01M phosphate). The mixture was then left under refrigerated conditions and stirred to equilibrate for 30 mins. The mixture was then washed thoroughly with further pH 0.1, 0.01M phosphate solution through a glass sinter funnel under reduced pressure. This fraction was kept as the acid volume. The process was then repeated using 0.025M phosphate (pH = 7.1) and 0.05M phosphate (pH = 7.1).

The results are illustrated in Table 5.

### SDS polyacrylamide Gel Electrophoresis

Using a BioRad mini gel system various papain and ficin preparations were separated using SDS PAGE. Polyacrylamide gels were run (at 200v) until completion. The gels were stained using Coomassie Brilliant Blue stain in glycerol and acetic acid. Very high protein loading was used ($0.4g.1^{-1}$) to produce the required resolution of the tested proteins.

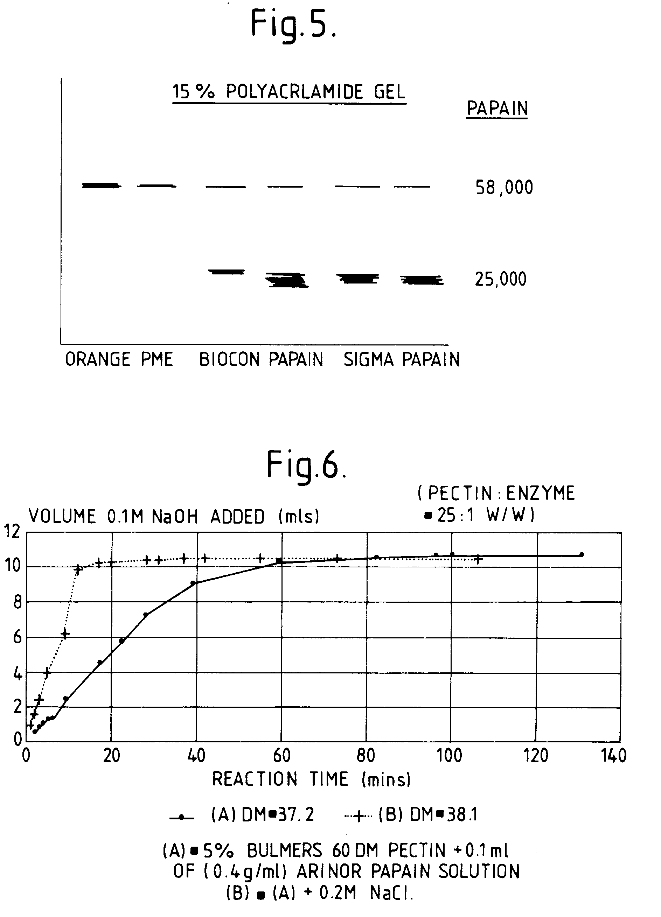

Schematic diagrams of the resulting gels are given in Figure 4 (ficin) and Figure 5 (papain). Figure 4 demonstrates the presence of a protein of molecular weight 61,000 in Sigma and Biocon ficin preparations, which is not present in pure ficin from Sigma. Figure 5 demonstrates the presence of a protein of molecular weight 58,000 in Sigma and Biocon papain preparations, corresponding to the sole resolved component of orange PME.

### Inhibition Tests

Bacterial and plant derived PMEs were tested for inhibition by N-acetyl-imidazole (NAI), a tyrosine selective inhibitor.

Inhibition by N-acetyl-imidazole was carried out in three different ways: by addition of the NAI to enzyme solution, to pectin solution, and to mixed enzyme and substrate. Typically we used 1% pectin (5% for papain also), enzyme concentrations of 0.005-0.088% and NAI concentrations of 1.4mg.100 ml.

Papain, ficin, bromelain and WGL derived PMEs were found not to be inhibited by NAI, while tomato, orange and bacterial PMEs are all inhibited by NAI.

In addition, both the ficin and papain PME activities were very stable when incubated with these proteases under conditions optimal for proteolysis.

### Examples

#### Example 1

5g of high methoxy pectin ex. Bulmers, Shropshire, UK (degree of methoxylation (DM) 70%) was dissolved in 500ml of deionised water at 50°C to give a 1% (w/v) solution (10 g dw $l^{-1}$). The pH of this solution was adjusted to pH 7.5 using 0.1M NaOH. A 0.2g/ml solution of ficin ex. Biocon (Quest Int., Crosshaven, Ireland) was prepared by dissolving ficin powder in deionised water. 2ml of this enzyme solution was added to the pectin solution, i.e. 4mls of enzyme solution/L of pectin solution, to give a working concentration of enzyme of 0.8g enzyme.$l^{-1}$ or 0.08g enzyme g pectin$^{-1}$. This sample was then incubated at 50°C while being gently agitated. During the reaction ester groups on the pectin are gradually hydrolysed to liberate methanol and form free carboxyl groups. Thus it is essential to maintain the pH constant. This is done by monitoring the pH of the reaction using a pH meter and adding 5OmM NaOH gradually during the reaction so as to
maintain the pH constant at pH 7.5. The rate of reaction is reached once no more NaOH is added, and the extent of demethoxylation achieved can be estimated from the total amount of NaOH added.

The final DM of the pectin can be determined more accurately by titration of the carboxyl groups. A sample is dried and 2g dw dissolved in water/conc. HCl/propan-2-ol(IPA)90/10/100, left for 15 minutes filtered, washed with 300ml IPA and filtered, washed with a second 300ml of IPA and filtered and then washed with absolute IPA. The precipitated pectin (0.5g) was then dissolved in deionised water, and titrated with 0.1M NaOH to pH 7.5 and the volume noted (a mls). 30ml of 0.1M NaOH is then added and the material is left stirring for 30 mins. Dilute $H_2SO_4$ equivalent to 30ml of 0.1M NaOH is added and is then titrated with 0.1M NaOH back to pH 7.5 and the volume noted (b mls). The degree of esterification is calculated as being 100 x b/a + b. In this case the original pectin had a DM of 60% and was reduced to a DM of 17.5% after 240 min incubation.

Similar experiments were carried out using other enzymes, and the results are summarised in Table 6.

Example 2

Further similar experiments were carried out using impure apple pectin, and the results are summarized in Table 7. The products of the reactions were tested to confirm that they gel with $Ca^{2+}$, confirming that the products are reasonably low in methoxy content and of fairly high molecular weight.

Example 3

In order to demonstrate that treatment of pectin with enzymes in accordance with the invention produces no significant reduction in pectin backbone length, molecular weight analysis of a Bulmers high methoxy pectin starting material before and after various demethoxylation treatments was carried out chromatographically. The chromatography system used employed a Gilson 305 pump, a Wyatt Technology Laser Photometer, a Waters 410 Differential Refractometer and Anachem gel permeation chromatography columns (guard, 6000, 5000 and 4000 arranged in series). Details of retention times are given in Table 8.

It can be seen that there is no significant difference between the measured retention times for the starting material before and after treatment with papain, ficin and orange PME, indicating no significant change in molecular weight and hence no significant undesired reduction in pectin backbone length. In contrast, treatment with current commercially available pectinase resulted in a substantial increase in retention time indicating a substantial reduction in molecular weight due to pectin backbone cleavage.

The following two examples concern experiments using crude papaya latex to simulate possible commercial reaction conditions. These examples use a higher pectin to PME ratio so as to minimise enzyme costs, while still having sufficient enzyme activity to achieve a DM of 30 to 40 in a short reaction time.

Example 4

20ml of 5% w/v Bulmers 60 DM citrus pectin was treated with 0.1 ml of PME (0.4g of Arinor papaya latex/ml) (i.e. lg pectin dw + 0.04g dw of latex). This is equivalent to substrate: enzyme ratio of 25:1. A final DM of 37.2 was obtained after 100 to 120 minutes reaction at 50°C and pH 7.5. The time course of the reaction is show by the solid line in Figure 6.

Example 5

In a comparable experiment, except that 0.2M NaCl was added, a final DM of 38.1 was obtained after 55 to 60 minutes reaction. The time course of the reaction is shown by the dotted line in Figure 6.

The PME is less than 10% of the crude latex papain and so when purified could be therefore used effectively at pectin to PME ratios of greater than 250:1.

TABLE 1

| PME ACTIVITIES | |
|---|---|
| Source | Activity (U/mg) |
| PAPAIN - ARINOR | 2.13 |
| PAPAIN - QUEST IRELAND/BIOCON (PROFIX EFL) | 0.68 |
| PAPAIN - SIGMA (PURE) | 0.0 |
| FICIN - QUEST- IRELAND/BIOCON | 0.11 |
| FICIN - PURE (SIGMA) | 0.0 |
| BROMELAIN - ARINOR | 0.021 |
| BROMELAIN - PURE (SIGMA) | 0.0 |
| WHEAT GERM LIPASE (SIGMA) | 0.07 |

TABLE 2

| $K_m$ OF PMEs | |
|---|---|
| ENZYME | $K_m(\text{mgml}^{-1})$, |
| FICIN (65% DM Substrate) | 3.48 |
| FICIN (27% DM Substrate) | 1.05 |
| PAPAIN (60% DM Substrate) | 18.37 |
| PLANT PMEs (Literature, 60-70% DM Substrate) | 0.4 - 2.4 |
| e.g. APPLE PME | 1.05 |

TABLE 3

| EFFECTS OF METHANOL AND SODIUM CHLORIDE ON PAPAIN PME | | |
|---|---|---|
| | PME ACTIVITY | |
| | 1% PECTIN | 5% PECTIN |
| PAPAIN | 0.42 | 1.85 (x 4.4) |
| PAPAIN + MEOH | 0.35 (x 0.83) | 1.78 (x 0.96) |
| PAPAIN + NaCl | 2.19 (x 5.21) | 2.15 (x 1.16) |
| PAPAIN + MEOH + NaCl | 2.01 (x 4.79) | 1.85 |

TABLE   4

COMPARISON OF PMEs PROPERTIES

| | papain PME | ficin PME | bromelain PME | WG lipase PME | Plant PME |
|---|---|---|---|---|---|
| pH optimum | 7.5 | 7.5 | 7.5 | 7.5 | 7.5 |
| temp. opt | 50°C | 50°C | 50°C | 37°C | 30°C |
| $K_m$ (mg/ml) | 19.7 | 2.3-4.7 | | 3.4 | 0.78[2] |
| Vmax (U) | 0.78 | 0.06-0.09 | | 0.06 | 0.61[2] |
| activity | ++(>1%) +(≤1%) | + | ± | + | +++ |
| inhibition by methanol | YES | NO | | | |
| stimulation by NaCl | YES | NO | | | YES |
| inhibition by impure pectin | YES | NO | | NO | |
| lowest DE citrus pectin | 31.9[3] | 28.9[4] ~20[5] | 42.0[4] | 43.5[4] | |
| lowest DE apple pectin | 40.6[3] | 23.2[3] | | | |
| active site: | | | | | |
| tyrosine | NO | NO | NO | NO | YES |
| cysteine | NO | NO | NO | NO | NO |
| serine | NO | NO | | | NO |
| stability to cysteine protease | YES | YES | | | YES[6] |

[2] literature value plant PME (Hagerman, A.E. + Austin, P.G. (1986) J. Agric. Food. Chem. 34, 440-444).

[3] (pectin)=1% (enzyme)=0.4% + NaCl

[4] (pectin)=0.25% (enzyme)=0.4%

[5] (pectin)=1% (enzyme)=0.8%

[6] tPME decrease of 50% after 5 days.

EP 0 580 252 A2

TABLE 5

PURIFICATION OF FICIN

| | Weight of protein (mg) | Activity[1] | Total Activity[2] | % Recovery of | | |
| --- | --- | --- | --- | --- | --- | --- |
| | | | | Protein | Protease Activity | PME Activity |
| Starting material | $1 \times 10^4$ | 43.5 | $4.35 \times 10^5$ | 100 | 100 | 100 |
| Void volume | $4.6 \times 10^3$ | 21 | $9.66 \times 10^5$ | 46 | – | 22.2 |
| Peak 1 | $1.02 \times 10^3$ | 15 | $1.5 \times 10^4$ | 10.2 | 25.5 | 3.4 |
| Peak 2 | $2.14 \times 10^3$ | 14.5 | $3.1 \times 10^4$ | 21.4 | 53.5 | 7.1 |
| Total recovered | 7.76 | – | $1.426 \times 10^5$ | 77.6 | 79 | 32.7[3] |

1   =   $H_1$ liberated $min^{-1} mg\ protein^{-1}$       ) PME activites

2   =   activity $min^{-1} mg\ protein^{-1} \times mg\ protein$      )

3   =   42.1% recovery allowing for 77.6% recovery of protein

TABLE 6

| CITRUS Enzyme: | m/a[1] | (Pectin) (% w/v) | (Enzyme) (% w/v) | Ratio (-) | Time (h) | final DE | ΔDE (-) | ΔDEx ratio/h |
|---|---|---|---|---|---|---|---|---|
| Ficin | m | 0.5 | 0.05 | 10 | 1.5 | 45.5 | 14.5 | 96.6 |
| | m | 0.5 | 0.1 | 5 | 1.75 | 34.7 | 25.3 | 72.3 |
| | m | 0.5 | 0.2 | 2.5 | 4 | 27.9 | 32.1 | 20.1 |
| | m | 0.5 | 0.4 | 1.25 | 2.33 | 19.0 | 41 | 22 |
| | m | 1 | 0.8 | 1.25 | 2.7 | 12.3 | 47.7 | 22.1 |
| | m | 1 | 0.4 | 2.5 | 2.15 | 17.0 | 43 | 50 |
| Ficin | m | 1 | 0.08 | 12.5 | 4 | 52.7 | 7.33 | 22.9 |
| | m | 1 | 0.32 | 3.125 | 4 | 28.3 | 31.7 | 24.7 |
| | m | 1 | 0.8 | 1.25 | 4 | 19.4 | 40.6 | 12.7 |
| | a | 1 | 0.4 | 2.5 | 1.88 | 29.3 | 30.7 | 40.8 |
| +NaCl | a | 1 | 0.4 | 2.5 | 1.42 | 33.3 | 26.7 | 47.0 |
| | a | 1 | 0.8 | 1.25 | 1.02 | 32.0 | 28.0 | 34.4 |
| | a | 0.25 | 0.4 | 0.625 | 0.65 | 28.9 | 31.1 | 29.9 |
| Papain | a | 1 | 0.4 | 2.5 | 2.05 | 36.7 | 23.3 | 28.4 |
| +NaCl | a | 1 | 0.4 | 2.5 | 0.85 | 31.9 | 28.1 | 82.7 |
| | a | 5 | 1 | 5 | 2.2 | 39.3 | 20.7 | 47.1 |
| | a | 5 | 0.6 | 8.33 | 1.53 | 47.3 | 12.7 | 68.9 |
| | a | 5 | 0.4 | 12.5 | 2.5 | 39.5 | 20.5 | 102.5 |
| | a | 4 | 0.2 | 20 | 2.92 | 40.9 | 19.1 | 130.8 |

| | | | | | | | | |
|---|---|---|---|---|---|---|---|---|
| | a | 4.5 | 0.2 | 22.5 | 3.35 | 39.1 | 20.9 | 140.4 |
| | a | 5 | 0.2 | 25 | 4.17 | 35.1 | 24.9 | 149.3 |
| | a | 5 | 0.2 | 25 | 4 | 39.3 | 20.7 | 129.4 |
| | a | 5.5 | 0.2 | 27.5 | 3.5 | 38.9 | 21.1 | 165.8 |
| | a | 6 | 0.2 | 30 | 2.77 | 39.4 | 20.6 | 223.1 |
| | a | 5 | 0.1 | 50 | 5.42 | 37.6 | 22.4 | 206.6 |
| | a | 1 | 0.04 | 25 | 4.2 | 56.7 | 3.3 | 19.6 |
| | a | 5 | 0.04 | 125 | 7.5 | 52.0 | 8 | 133.3 |
| | a | 5 | 0.04 | 125 | 6.33 | 42.0 | 18 | 354 |
| WG lipase | a | 1 | 0.4 | 2.5 | 0.68 | 43.5 | 16.5 | 60.7 |
| Bromelain | a | 1 | 0.4 | 2.5 | 2 | 42.0 | 18 | 22.5 |

[1] manual or on autotitrator

EP 0 580 252 A2

TABLE 7

IMPURE APPLE PECTIN

| Enzyme e | m/a[1] | (Pectin) (% w/v) | (Enzyme) (% w/v) | Ratio (-) | Time (h) | final DE | ΔDE (-) | ΔDExratio /h |
|---|---|---|---|---|---|---|---|---|
| Ficin | m | 1[a] | 0.4 | 2.5 | 3.15 | 17.0 | 62.0 | 49.2 |
| | m | 1[b] | 0.4 | 2.5 | 3.15 | 17.5 | 42.5 | 33.7 |
| | m | 1[c] | 0.4 | 2.5 | 0.8 | 12.0 | 35.0 | 109.4 |
| Ficin | a | 0.25[b] | 0.4 | 0.625 | 0.85 | 23.1 | 36.9 | 27.1 |
| | a | 2.5[b] | 0.4 | 2.5 | 4.833 | 16.8 | 43.2 | 22.4 |
| | a | 0.25[d] | 0.4 | 0.625 | 1.133 | 23.3 | 47.7 | 26.3 |
| | a | 1[d] | 0.4 | 2.5 | 3.333 | 26.0 | 45.0 | 33.8 |
| | a | 2.5[d] | 0.4 | 6.25 | 7.45 | 32.8 | 38.2 | 32.1 |
| Papain | a | 0.25[d] | 0.4 | 0.625 | 0.87 | 40.6 | 19.4 | 13.9 |

[1] manual or on autotitrator

[a] Biocon '91 DE = 79%                    )
[b] Biocon '85 DE = 60%                    )
[c] Biocon (India) '85 DE = 47%           )   Impure pectin substrates
[d] Herbstreith & Fox DE = 71%            )
   (dried apple residue)

EP 0 580 252 A2

TABLE 8

| MOLECULAR WEIGHT ANALYSIS OF ENZYME-TREATED PECTINS | |
|---|---|
| | Retention Time (mins) |
| Starting material * | 41.5 |
| Sigma Papain Treated | 42.5 |
| Sigma Ficin Treated | 44.5 |
| Sigma Orange PME Treated | 43.0 |
| Commercial Pectinase Treated | 73.5 |

\* Bulmers High Methoxy Pectin

**Claims**

1. An enzyme having pectin methyl esterase activity not inhibited by tyrosine selective inhibitors.

2. An enzyme having pectin methyl esterase activity, that is stable to cysteine proteases.

3. An enzyme according to claim 1-2, having a molecular weight in the range about 58,000 to about 61,000.

4. An enzyme according to claim 1, 2 or 3, that has no significant endoglycosidase activity.

5. An enzyme according to any one of the preceding claims, obtainable from plant sources including papaya sap, fig sap, bromelard sap and wheat germ.

6. A method of reducing the degree of methoxylation of pectin, comprising treating pectin with an enzyme in accordance with any one of the preceding claims.

7. A method according to claim 6, wherein pectin is treated with enzyme in the form of an extract from papaya, fig, bromelard and/or wheat germ.

8. A method according to claim 7, wherein pectin is treated with ficin, papain, bromelain and/or wheat germ lipase.

9. A method according to claim 6, 7 or 8, wherein the pectin is at a concentration of 10% (w/v) or less.

10. A method according to any one of claims 6 to 9, wherein the extent of demethoxylation is controlled by varying the amount of enzyme added.

11. A method according to any one of claims 6 to 10, wherein an alkaline solution is added at a suitable rate to maintain a pH of between 6 and 9.

12. A method according to any one of claims 6 to 11, comprising initial treatment with enzyme obtainable from papaya followed by treatment with enzyme obtainable from fig.

13. A method according to any one of claims 6 to 12, wherein the pectin comprises pectin from apple and/or citrus sources.

14. Low methoxy pectin having neutral sugar side chains.

15. A low methoxy pectin according to claim 14 prepared from a gum by demethoxylation, characterized in that it has retained at least 60% of the neutral sugar side chains after demethoxylation when compared with the original gum.

Fig.1.

PME ACTIVITY (u/mg ENZYME)

- PAPAIN 0.02%
+ FICIN 0.1%
* FICIN 0.04%
□ WG LIPASE 0.04%

PECTIN CONCENTRATION (%)

Fig.2.

AMOUNT OF 0.1M NaOH ADDED (ml)          FINAL DM

29.3

36.7

* 43.5

□ 42

TIME (min)

- FICIN
+ PAPAIN
* WG LIPASE
□ BROMELAIN

1% PECTIN
0.4% ENZYME
RATIO 2.5:1

## Fig.3.

FINAL DE (%)

RATIO (mg PECIN / mg ENZYME)

- ■ FICIN (PURE PECTIN)
- + PAPAIN (PURE PECTIN)
- \* FICIN (BIOCON '85)
- □ FICIN (H & F)          } CRUDE / UNPURE
- △ PAPAIN (H & F)

## Fig.4.

15 % POLYACRYLAMIDE GEL

FICIN

61,000

26,500
26,000

PURE FICIN          BIOCON FICIN     SIGMA FICIN
(SIGMA)

# Fig.5.

15 % POLYACRLAMIDE GEL

PAPAIN

58,000

25,000

ORANGE PME    BIOCON PAPAIN    SIGMA PAPAIN

# Fig.6.

( PECTIN : ENZYME
■ 25 : 1  W / W )

VOLUME 0.1M NaOH ADDED (mls)

REACTION TIME (mins)

─▲─ (A) DM■37.2    ─+─ (B) DM■38.1

(A)■5%  BULMERS 60 DM PECTIN + 0.1 ml
OF ( 0.4 g/ml) ARINOR PAPAIN SOLUTION
(B) ■ (A) + 0.2M  NaCl.